Europäisches Patentamt

European Patent Office

Office européen des brevets

(11) Veröffentlichungsnummer: **0 164 423**
**A2**

# EUROPÄISCHE PATENTANMELDUNG

(21) Anmeldenummer: 84105554.4

(22) Anmeldetag: 16.05.84

(51) Int. Cl.⁴: **A 61 K 47/00**

(30) Priorität: 09.05.84 DE 3417022

(43) Veröffentlichungstag der Anmeldung:
18.12.85 Patentblatt 85/51

(84) Benannte Vertragsstaaten:
AT BE CH FR GB IT LI LU NL SE

(71) Anmelder: Theurer, Karl Eugen, Prof.Dr.med.
Brunnwiesenstrasse 23
D-7302 Ostfildern 1(DE)

(72) Erfinder: Theurer, Karl Eugen, Prof.Dr.med.
Brunnwiesenstrasse 23
D-7302 Ostfildern 1(DE)

(54) Träger- und Begleitstoffe für biologische Wirk - und Regulationsfaktoren aus Zellen und Geweben und deren Anwendung.

(57) Die Effektivität der Anwendung von pflanzlichen und tierischen Wirkstoffen -insbesondere Hormonen, Regulationsfaktoren, Überträgerstoffen, Enzymen, ebenso von Vitaminen und Spurenelementenwird verbessert, indem diese an Polypeptide, Peptide und bzw. oder Nukleotide als Träger-und Begleitstoffe gebunden werden, die man aus mikrobiellen, pflanzlichen und bzw. oder tierischen Makromolekülen durch chemische Hydrolyse oder enzymatischen Abbau gewinnt. Die Alkalisierung der beladenen Trägerund Begleitstoffe - unter Mitverwendung von Spurenelementen und möglicherweise von oberflächenaktiven Substanzenwirkt Umweltschädigungen der Flora entgegen. Dabei können auch energieübertragende Systeme mitverwendet werden. Das "Waldsterben" infolge Adaptationsschwierigkeiten an Umweltsveränderungen ist ein bevorzugtes Anwendungsgebiet, ebenso aber auch die Verbesserung der Ausnutzung und Verwertung von Futtermitteln und der Resistenz gegen Infektionen und Stress in der Aufzucht von Nutztieren und die Prophylaxe und Therapie von Schäden durch ionisierende Strahlen und zytotoxische Substanzen. Die Wirksamkeit von Arzneimitteln der Human und Veterinärmedizin wird verbessert und protrahiert. In der Bakteriologie werden die Präparate zur Stimulierung des Wachstums von Mikroorganismen und in der Gewebezüchtung zur Verbesserung des Angehens von Zellkulturen sowie zur Erhaltung der Spezifität und der Zellteilungskapazität den Nährmedien zugesetzt.

EP 0 164 423 A2

0164423

Träger- und Begleitstoffe für biologische Wirk-
und Regulationsfaktoren aus Zellen und Geweben
und deren Anwendung

Beschreibung:

Aufgrund des Vorurteils, daß Begleitstoffe sich
nachteilig auswirken könnten, besteht in der
Pharmakologie die Tendenz zur Monotherapie mit
isolierten Wirkfaktoren. Die experimentelle Empirie zeigt jedoch eine höhere Effektivität von
natürlichen multifaktoriellen Wirkstoffgemischen
als von isolierten Einzelfaktoren (vergl. K. E.
THEURER: Pharmakologie: Eingliederung der Therapie mit makromolekularen Organextrakten in die
moderne Pharmakologie: DER KASSENARZT 21, 12
(1981). Unveränderte Naturstoffe müssen dabei
vom Empfängerorganismus zum großen Teil in Untereinheiten und -Bestandteile zerlegt werden, bevor
sie zur Wirkung gelangen. Nicht unmittelbar wirksame Bestandteile werden verstoffwechselt und weiter
abgebaut. Sie dienen z. T. auch als Reparaturmaterial für defekte Strukturen, wie auch als Transportvehikel und Depotstoffe.

Durch vorliegendes Verfahren werden die biologischen
Träger- und Begleitstoffe in eine direkt wirksame
Form vor ihrer Anwendung zusammengefügt. Es handelt sich dabei um künstliche Abbauprodukte von
Eiweiß und Nukleinsäuren aus biologischen Zellen
und Geweben, insbesondere aus nicht löslichen, korpuskulären und Struktur-Bestandteilen, aus aphathogenen Mikroorganismen und bzw. oder tierischen und

0164423

bzw. oder pflanzlichen Geweben. Als Ausgangsmaterial kann der nicht wasserlösliche Rückstand von einer primären Aufschließung und Extraktion der Naturstoffe dienen. Rückstände aus der Zentrifugation oder Filtration werden üblicherweise verworfen. Die erfindungsgemäße Weiterverwendung bedeutet deshalb auch einen wirtschaftlichen Nutzen, jedoch können auch direkt native Zellen und Gewebe verwendet werden. Isolierung und Abbau von hochmolekularem, nicht löslichem Eiweiß und Nukleinsäuren entsprechen dem Stand der Technik (vergl. Biochem. Taschenbuch: H. M. RAUEN: Springer Verlag). Isolierung und Abbau erfolgen in verschiedenen Stufen, gegebenenfalls nach Fettextraktion durch nicht denaturierende Fettlösungsmittel, chemische Hydrolyse, wie auch enzymatisch bis zu Polypeptiden, Peptiden und Nukleotiden mit einem Mol. Gew. zwischen 500 und 50 000 Dalton. Kleinere Bruchstücke können mitverwendet oder aber durch Dialyse, Gelfiltration oder chromatographische Verfahren ohne Erhitzung separiert werden. Zunächst werden die Polysaccharide, dann die Eiweiße und schließlich die Nukleinsäuren zur gewünschten Größenordnung abgebaut. Zum enzymatischen Abbau werden trägergebundene Enzyme (Proteasen, z. B. Pepsin, Papain, Bromelin u. a.; Restriktionsenzyme, DNasen, RNasen) bei ihrem jeweiligen $p_H$-Wirkstoffoptimum verwendet und nach getaner Wirkung durch Zentrifugation oder Filtration für eine erneute Verwendung zurückgewonnen. Beim stufenweisen Abbau muß jeweils das optimale $p_H$ für die verwendeten Enzyme durch Pufferzusätze eingestellt werden. Als Trägersubstanz für Enzyme eigenen sich Kolloide und Partikel aus Zellulose (Sephadex), Kollodium und anderen Kunststoffen.

- ₹-

0164423

Die Peptidmischung sollte die Aminosäuren Alanin, Asparaginsäure, Cystein, Cystin, Glutaminsäure, Methionin, Phenylalanin, Thyrosin und Tryptophan enthalten. Nach der Fragmentierung können Peptide und Nukleotide getrennt voneinander oder aber auch gemischt als Träger- und Begleitstoffe verwendet werden. Der amphothere Charakter der Peptide und Nucleotide erlaubt kovalente, wie auch adsorptive Bindungen mit den zu konjugierenden biologischen Wirkstoffen, jedoch sind auch besondere chemische Konjugationsverfahren, wie sie insbesondere zur Gewinnung von konjugierten Antigenen verwendet werden (vergl. H. SCHMIDT: Fortschritte der Serologie, Verlag Dietrich Steinkopff) möglich.

Die Träger- und Begleitstoffe können für alle Arten von Arzneimitteln, insbesondere für biologische Arzneimittel, Pflanzen- und Organextrakte, Hormone, Überträgerstoffe, Biological Response Modifiers, Redox- und Energie-übertragende Systeme und bzw. oder Enzymen verwendet werden.

Zur Beeinflussung der Fora, von Bäumen und Pflanzen, werden Pflanzenhormone bzw. Wuchsstoffe (Auxine), die auch synthetisch gewonnen sein können, einzeln oder in Kombination verwendet. Phytokine werden besonders in Verbindung mit Nucleotiden eingesetzt. Die Indolessigsäure-Derivate, Gibberelline und Abscisinsäure werden bevorzugt mit Peptiden konjugiert. Es kann jedoch auch eine Kombination mit Peptiden und Nucleotiden entsprechend den jeweiligen Bedürfnissen erfolgen. Auch richtet sich bei Kombinations-Präparaten die Relation der verschiedenen Wirkfaktoren nach ihren Funktionen und ihrem
Wechselspiel. Z. B. darf die Abscisinsäure, die
einen Verschluß der Spaltöffnung bei Pflanzen
bewirkt, für die hormonelle Regulation welkender Pflanzen nur einen Bruchteil der anderen
Wuchsstoffe betragen.

Je nach $p_H$ des Bodens und der Übersäuerung durch
Umweltsschäden werden die Konjugate auf ein mehr
oder weniger alkalisches $p_H$ zwischen 7,1 und 7,8
durch Zusatz geeigneter Puffersysteme oder alkalisierender Bi-Carbonate, -Phosphate oder Citrate
der Erdalkalien eingestellt. Dabei ist die $p_H$-
Veränderung durch höhere Verdünnung mit Wasser
bei der Anwendung zu beachten. Eine Mitverwendung von Spurenelementen, insbesondere von Mangan,
Magnesium, Zink, Kupfer und Eisen, kann je nach
Art der Böden und der Umweltsbedingungen zweckmäßig sein. Auch dabei kann die Zusammensetzung
variieren.

Die Herstellung eines wasserlöslichen Trockenpulvers, das vor der Anwendung in geeignetem Verdünnungsverhältnis in Wasser gelöst wird, ist
zweckmäßig. Dabei ist auch auf die Salzkonzentration zu achten. Auch können fertig hergestellte Lösungen verwendet werden. Sonst erfolgt die Herstellung von Lösungen aus dem
Trockenkonzentrat oder konzentrierten Lösungen
nach Gebrauchsanweisung, eventuell mit Bestimmung
des $p_H$.

0164423

Die Anwendung in der Flora kann durch Besprühen des Laubes, wie durch Begießen mit Lösungen oder durch Bestreuen des Bodens mit Trockenpulver erfolgen. Auch ein Zusatz zu Wasserkulturen ist möglich. Indikationen sind die Resistenzsteigerung der Flora, insbesondere von Weltwirtschafts- und Nutzpflanzen, Bäumen, Sträuchern und anderen Pflanzen gegen ungünstige Klima- und Milieu-Einflüsse, Umweltsschäden, Pflanzenschädlinge, zur Beschleunigung des Wachstums und der Verkürzung der Reifungszeit, zur Ertragssteigerung, Erleichterung der Mutations- und Kreuzungsbereitschaft, wie auch zur Kräftigung. Das bevorzugte Indikationsgebiet sind Umweltsschäden durch sogenannten "sauren Regen" und Umweltsverschmutzung, die zum "Waldsterben" führen. Dabei ist die Relation der Konzentrationen der verschiedenen Pflanzenhormone und das alkalische $p_H$ besonders zu beachten. Das Verhältnis von Abscisinsäure und den anderen Pflanzenhormonen sollte nicht unter 1 : 100 betragen. Indolessigsäure soll zum Besprühen in einer Konzentration von ng bis pg / ml, zum Düngen im mg- bis µg-Bereich verwendet werden. Eine Mitverwendung von Redox- und Energie-übertragenden Systemen (NAD, NADP und bzw. oder ATP), einzeln oder in Kombination mit den Wuchsstoffen, ist möglich. Die Konzentration des Peptid-Nucleotid - Gemisches beträgt das 10 bis 1000-fache der verwendeten Wirkfaktoren. Eine Verdünnung bei der Düngung kann durch Einmischen des Trockenkonzentrates in Kompost oder Torf erfolgen.

Für die Anwendung bei Menschen und Tieren können ebenfalls geeignete Hormone an die Trägerstoffe konjugiert werden. Insbesondere eignen sich dazu

0164423

sogenannte Gewebehormone, wie auch alle anderen menschlichen und tierischen Hormone. Durch die Konjugation wird die direkte Verfügbarkeit der Hormone bei der Anwendung verringert und die Ansprechbarkeit erhöht. Dabei kommt eine protrahierte Depotwirkung zustande. Für die Konjugation mit Träger- und Begleitstoffen eignen sich auch Wirkfaktoren aus Zellen und Geweben, die in einem speziellen vorausgehenden Extraktionsvorgang gewonnen werden, wie z. B. von sogenannten "Biological Response Modifiers", Abwehrstimulatoren, Paramunitätsinducern, Überträgerstoffen, z. B. Neurotransmitter, Endorphinen, Encephalinen, Interferone, Prostaglandine und anderer Regulationsfaktoren sowie Redox- und Energie-übertragende Systeme, Enzyme, die die Trägersubstanzen nicht weiter abbauen. Ebenso ist es aber auch möglich, Hemmsubstanzen bestimmter Wirkfaktoren, insbesondere Antihormone oder Antienzyme, zu konjugieren. Das $p_H$ solcher Produkte sollte auf etwa 7,4 eingestellt werden. Die Konzentration der Wirksubstanzen beträgt zwischen 1:10 bis 1:10000 der Trägersubstanz. Das Produkt kann auch hier als lösliches Trockenpräparat oder als wässrige Lösung hergestellt werden.

Die Präparate besitzen bei Mensch und Tier keinerlei nachteilige Nebenwirkungen. Bei Verwendung von Molekulargewichten bis zu etwa 3000 Dalton sind auch keine nachteiligen Sensibilisierungsvorgänge zu befürchten. Sonst könnten diese durch immunologisch-tolerogene Dosierung in ansteigender Konzentration und kurzen Intervallen, beginnend mit sehr hohen Verdünnungen, verhindert werden. Die Anwendung bei Tieren kann über Futtermittel, denen sie beigemischt werden, oder über die Wasseraufnahme erfolgen. Es wurde festgestellt, daß
selbst Tiere, die mit Anabolica- und Antibiotica-Zusätzen gefüttert wurden, eine weitere Wirkungssteigerung bei zusätzlicher Anwendung solcher Präparationen zeigten; die mit Organextrakt-
Zusätzen gefütterten Tiergruppen überdies gesund
blieben und keine nachteiligen Verhaltensweisen
zeigten. Demgegenüber kam es bei Kontrollgruppen
zu Ausfällen durch Grippe, Husten, Fellbeißen
und bei Hühnern durch Federpicken. Auch traten
keine Herz- und Kreislauftodesfälle mehr auf,
für die Schweine besonders empfindlich sind.
Für die menschliche Ernährung bedeuten Zusätze
dieser Präparate zum Tierfutter keinerlei Gefährdung, weil alle Faktoren verstoffwechselt
werden und keine Rückstände bilden. Die Anwendung erfolgt in einer Endverdünnung von mg bis
ng mit einem Wirkstoffgehalt von µg bis pg. Es
hat sich bewährt, gleichzeitig eine oberflächenaktive Substanz beizumischen, z. B. Natriumlaurylsulfat in einer Endkonzentration von 10 µg. Dadurch wird die Resorption und Verteilung im Organismus erleichtert. Dasselbe gilt auch für die
Verwendung von wässrigen Verdünnungen.

Solche Präparationen eignen sich auch zur Anwendung als Arzneimittel und Diätetica bei
Menschen. Aufgrund der Verwendung verschiedenartiger Wirkstoffe sind unterschiedliche Indikationen möglich. Hervorzuheben ist die Prophylaxe
und Therapie von Schäden durch ionisierende Strahlen
und Zytostatika. Bei Bestrahlen mit einer LD 50
werden dabei in Kombination mit zytoplasmatischen Organextrakten (NeyTumorin [R] der vitOrgan Arzneimittel GmbH., 7302 Ostfildern - Ruit) die Überlebensrate auf 100 % gesteigert (vergl. P. SCHICK:
Strahlenschutzsubstanzen auf zytoplasmatischer
Basis (Revitorgan [R]) im Test mit letalen Strahlendosen: THERAPIEWOCHE, Sonderausgabe 33, 21 A, 1983.
Auch in der Onkologie erscheint der Einsatz solcher
Präparate besonders zur Prophylaxe, eventuell als
Diäteticum, aussichtsreich.

Die Präparationen lassen sich zur Erleichterung und
Verbesserung der Anzüchtung von humanen und tierischen
Zell- bzw. Gewebe-Kulturen sowie zur Erhaltung des
Spezifitätsgrades und zur Stimulierung erschöpfter
Zellkulturen verwenden. Bakterienkulturen können
ebenfalls in entsprechender Weise günstig beeinflusst werden. Es müssen dabei Wirkfaktoren aus
analogen Spezies mit den Träger- bzw. Begleitsubstanzen, bei optimalem $p_H$ und Ionenkonzentration,
dem Nährmedium zugesetzt werden.

Erfindungsgemäß war die Verwendung von Träger- bzw.
Begleitstoffen für biologische Wirkfaktoren, in Verbindung mit $p_H$-Einstellung und Mitverwendung oberflächenaktiver Substanzen, bisher unbekannt. In der
deutschen Patentschrift 2249697 vom 11.10.1972 werden als Bestandteil sonst üblicher Futtermittel
Stoffe verwendet, die bei einem Verfahren zur gesteuerten chemischen Aufschließung von Organen für
therapeutische Zwecke, unter Verwendung von Dämpfen
chemischer Reagenzien, erhalten worden sind. Dieses
Verfahren kann auch zur Gewinnung der beanspruchten
Träger- und Begleitstoffe, wie auch zur Gewinnung
der biologischen Wirkstoffe verwendet werden. Die

0164423

Trägerstoffe werden durch weitere Verarbeitung der Rückstände nach Extraktion gewonnen. Dies bedeutet eine verbesserte Ausnützung der Ausgangssubstanzen und birgt nicht zuletzt auch ökonomische Vorteile in sich. Der grundsätzliche Unterschied besteht aber darin, daß bei vorliegendem Verfahren Träger- und Begleitsubstanzen separat gewonnen und dann sekundär mit Wirkfaktoren konjugiert werden.

Die DPA 2110683 vom 5.3.1981 ist ein Anwendungsverfahren zur Resistenzsteigerung, Beschleunigung des Wachstums, Verkürzung der Reifungszeit, Ertragssteigerung, Erleichterung von Mutationen und Kreuzungsbereitschaft sowie Kräftigung von Teilen der Flora und Aktivierung von Nutz- und Heilpflanzen-Säften. Auch dort werden keine entsprechenden Träger- bzw. Begleitsubstanzen für Wirkstoffe verwendet. Saatgut oder Pflanzenteile werden mit Extrakten aus gesunden und bzw. oder pathologischen tierischen Geweben und bzw. oder Mikroorganismen und bzw. oder aus Pflanzen behandelt. Die verwendeten Extrakte werden nach bekannten Verfahren aus den genannten biologischen Geweben hergestellt, desgleichen auch Fraktionen, die man aus den Extrakten gewinnt, insbesondere Lipide, Polysaccharide, Proteide, Proteine und die verschiedenen Nucleinsäuren. Demnach bestehen die Extrakte nicht aus Polypeptiden, Nukleotiden, die als Träger- bzw. Begleitstoffe verwendet werden können. Es wäre dazu auch ein zweiter Arbeitsgang der Konjugation erforderlich. Auch wird keine Abpufferung beansprucht. Die Konjugation von zusätzlichen Wirkstoffen erfolgt an hochmolekulare Stoffe, die bei vorliegendem Verfahren nicht zur Anwendung kommen. Auch hier bedeutet das Verfahren einen ökonomischen Vorteil, weil die Ausgangsstoffe besser genutzt und die Bestandteile und Untereinheiten von Makromolekülen biologisch unmittelbar verwertet werden, was zu einer besseren Effektivität führt.

Beispiel 1

Zur Vorbeugung und Behandlung von Schäden an Bäumen und Pflanzen durch Milieu- und Umwelts-Einflüsse (Waldsterben) werden als Träger- und Begleitstoffe Polypeptide und Nucleotide verwendet, für die als Ausgangsmaterial Pflanzen und tierische Gewebe bzw. Blut dienen. Die Aufschließung von Zellen bzw. Degradierung von Makromolekülen erfolgt nach bekannten schonenden biochemischen Methoden (vergl. Biochemisches Taschenbuch: H. M. RAUEN: Springer Verlag), bei denen die Spezifität der Untereinheiten und Bestandteile erhalten bleibt. Die Gewebe werden möglichst im tiefgekühlten Zustand gemahlen oder auch frisch bei Temperaturen unter 56° C in nahezu isotoner physiologischer Lösung homogenisiert, wobei das Mischungsverhältnis von Ausgangsmaterial und Lösungsmittel festgelegt ist. Zur Entfernung von Lipiden kann diese Homogenisierung auch in Fettlösungsmitteln, z. B. Petroläther erfolgen, aus dem nach Dekantierung die Lipidfraktion gewonnen wird, während die Rückstände in wässrige Lösungsmittel zur Weiterbehandlung aufgenommen werden. Die gesamte Aufarbeitung von pflanzlichen und tierischen Ausgangsstoffen kann getrennt oder aber auch bei Mischungen erfolgen. Die löslichen Faktoren des Homogenats werden zur weiteren Aufarbeitung auf Wirkfaktoren durch Zentrifugieren und bzw. oder Filtrieren entfernt und der Rückstand mit Säure bzw. Alkalilösungen aufgeschlossen. Nach erfolgter Säure-Hydrolyse, insbesondere von Polysacchariden und Zellulose, wie auch von Proteiden und Proteinen, werden Enzyme eingesetzt. Bei stark saurem $p_H$ wird zunächst Pepsin zum Abbau von Proteinen verwendet, bei Alkalisierung Trypsin. Es können jedoch auch andere proteolytische Enzyme eingesetzt werden, wobei das $p_H$-Optimum für die Enzymwirkung durch Alkalisierung bzw. Ansäuerung eingestellt wird. Zusätzlich können Harnstoff und bzw. oder oberflächenaktive Substanzen, insbesondere Natriumlaurylsulfat, mitverwendet werden. Danach wird erneut zentrifugiert bzw. filtriert und die Polypeptid- bzw. Peptidfraktion gewonnen. Der Rückstand wird mit RNasen bzw. DNasen oder Restriktionsenzymen zu Polynucleotiden und Nucleotiden abgebaut. Auch hier können bei optimalem $p_H$ oberflächenaktive Substanzen oder Harnstoff mitverwendet werden. Zum enzymatischen Abbau werden die Enzyme an kolloidale oder korpuskuläre Trägerstoffe, z. B. aus Zellulose (Sephadex), Kollodium oder andere geeignete Kunststoffe gebunden, die es erlauben, die Enzyme durch Zentrifugation rückzugewinnen und erneut wieder zu verwenden. Auch können für die enzymatische Auftrennung chromatographische Verfahren verwendet werden, die eine Wiederbenützung der Enzyme ermöglichen. Die erhaltenen Polypeptide und Peptide und andererseits die Nucleotide werden nun getrennt für sich oder als Mischung erfindungsgemäß verwendet oder zur späteren Verwendung in Lösungen oder als Trockenpulver konserviert.

- 12-

Zur Bekämpfung des Waldsterbens und Stärkung der Widerstandskräfte der Flora durch Substitution von biologischen Faktoren und Pharmaka wird eine Mischung von Abscisinsäure (1 Teil) mit β-Indolessigsäure oder α-Naphthylessigsäure (1000 Teile), Gibberilinsäure (100 Teile) und Cytokinine (10 Teile) sowie Nicotinamid-adenin-dinucleotid und bzw. oder das entsprechende Phosphat (100 Teile), möglicherweise auch Adenosintriphosphat (je 10 - 100 Teile) an $10^4$- bis $10^6$-Teile der Nucleotid-Polypeptidmischung gebunden. Aufgrund der amphotären Eigenschaften der Träger- bzw. Begleitstoffe ist eine Salzbildung möglich, sonst können auch bekannte chemische Konjugationsmethoden, wie Diazotierung, das Oxazolan-Verfahren in Verbindung über Isozyanate, Carbobenzoxy-Verbindungen und andere verwendet werden. Bei Übersäuerung der Umwelt bzw. der Böden werden zur Neutralisierung den Präparationen geeignete chemische Puffersysteme zugesetzt, die das $p_H$ zwischen 7,2 bis 7,8 einstellen oder entsprechende Bicarbonate, -Phosphate oder Citrat von Kalium, Natrium oder Aluminium sowie geeignete Spurenelemente von Mn, Mg, Zn, Cu bzw. Eisen zugesetzt. Ebenso können auch oberflächenaktive Substanzen mitverwendet werden. Die Mischung der Trockenpulver kann direkt zur Einmischung in Torf oder Kompost oder zum Bestreuen des Bodens als Düngermittel verwendet werden. Zum Besprühen des Laubs oder der Nadeln der Pflanzen bzw. Bäume werden Lösungen hergestellt, mit einem Gehalt von mg bis μg der Mischung in wässrigen Lösungen mit $p_H$ zwischen 7,1 und 7,8.

Beispiel 2

Zur Anregung des Wachstums und der Gewichtszunahme von Nutztieren werden Polypeptide, Peptide und Nucleotide, die analog dem Beispiel 1 gewonnen wurden, als Trägerstoffe für biologische Wirkstoffe verwendet, die nach DP 2249697 gewonnen werden. Diese dienen als Zusatz zu den sonst üblichen Futtermitteln oder können auch über die Wasseraufnahme zugeführt werden. Das Gewichtsverhältnis von Wirkstoffen und Trägerstoffen beträgt $10^{-3}$ bis $10^{-4}$. Bei einer Dosierung des Fertigproduktes im Mikrogrammbereich pro Gramm Futtermittel bzw. Milliliter Wasser der Präparation können anabole Hormone, Vitamine, Elektrolyte und Spurenelemente sowie auch oberflächenaktive Substanzen zugesetzt werden. Die Verwendung der Trägerstoffe ermöglicht eine Einsparung der zugesetzten biologischen Wirkstoffe und bedeutet eine wesentliche Verbesserung des DP 2249697.

Beispiel 3

Verwendung der Trägersubstanz nach Beispiel 1 zur Prophylaxe und Therapie von Schäden durch ionisierende Strahlen oder zytotoxische Einwirkungen. Hier werden die Träger- bzw. Begleitsubstanzen für zytoplasmatische Organextrakte, insbesondere aus dem maternen Anteil der Placenta, Thymus, Leber,Knochenmark -eventuell als Einzelorganextrakte, oder als Kombinationen-, insbesondere wie sie in dem Präparat NeyTumorin $^{(R)}$ vorliegen, verwendet. Das Gewichtsverhältnis der Träger- bzw. Begleitsubstanzen zu den zytoplasmatischen Organ-

extrakten beträgt $10^2$ bis $10^3$ / g, die Anwendungskonzentration mit der Präparation $10^{-6}$ bis $10^{-9}$ g/ml bei i.m.- oder i.v.-Injektionen, z. B. nach dem Strahlenschaden 4 Stunden, 8 Stunden, 2 Tage, 6 Tage, 11 Tage und 14 Tage. Die Behandlung wirkt sich auf eine raschere Wiederherstellung der Leukozytenzahl und eine signifikante Steigerung der Überlebensrate aus.

Beispiel 4

Verwendung der Träger- und Begleitsubstanz nach Beispielen 1 - 3 in der Human- und Veterinärmedizin, in Verbindung mit speziellen organotherapeutischen Wirkstoffen (vergl. Zytoplasmatische Organo-Therapie nach THEURER) und zytotoxischen bzw. zytostatischen Pharmaka in der Onkologie. Hier beträgt das Mischungsverhältnis der Wirkstoffe zu den Trägerstoffen 1 : 100 bis 1 : 10000. Die Verwendung der Trägerstoffe und zytoplasmatischen Organfaktoren verbessert die Verträglichkeit zytotoxischer bzw. zytostatischer Substanzen und erlaubt eine vielfach höhere Dosierung. Die Resistenz gegen Infektionen wird gesteigert und die Lebensqualität erhalten bzw. verbessert.

Beispiel 5

Verwendung der Träger- und Begleitstoffe nach Beispielen 1 - 3, für die Konjugation mit Wirkstoffen aus Organextrakten, Vitaminen, Hormonen, Spurenelementen und Elektrolyten als Zusatz zum Nährmedium von Zell- und Gewebekulturen, bei mindestens gleichem Effekt auf die Verbesserung des Angehens der Zellen und Gewebe bei der Anzüchtung, auf den Synthesestoffwechsel und auf die Stabilisierung. Gegenüber Entdifferenzierung bzw. der Beeinflussung der Proliferationsfähigkeit ermöglicht die konjugierte Anwendung der Wirkstoffe mit Trägerstoffen eine Einsparung an Wirkstoffen.

Beispiel 6

Verwendung der Träger- und Begleitstoffe nach Beispielen 1, 2 und 5. Es werden dabei jedoch Bakterienwuchsstoffe und Wirkfaktoren aus Bakterienextrakten mit den Trägerstoffen konjugiert. Die Trägerstoffe selbst können aus Massenzüchtungen von Mikroorganismen gewonnen werden. Als Nährmedien für die Gewinnung der Bakterienmassen zur Herstellung der Trägerstoffe können dabei Rückstände der Extraktion von Pflanzen und Tiergewebe bzw. -Zellen dienen. Die konjugierten Träger- und Begleitstoffe werden zur Stimulierung des Wachstums in Bakterienkulturen in Konzentrationen von µg bis pg zugesetzt.

— 16-

Träger- und Begleitstoffe für biologische Wirk- und Regulationsfaktoren aus Zellen und Geweben und deren Anwendung

Patentansprüche:

1. Träger- und Begleitstoffe für biologische Regulationsfaktoren und Pharmaka, dadurch gekennzeichnet, daß hoch- und makromolekulare Bestandteile aus biologischen Zellen (Mikroorganismen und bzw. oder tierischen oder pflanzlichen Geweben) sowie Blut, insbesondere nicht lösliche Rückstände aus Extrakten durch chemische und bzw. oder enzymatische Spaltung und Beseitigung von Lipiden, Zellulose und Polysacchariden in Polypeptide, Peptide und Nucleotide mit einem Molekulargewicht von 500 bis 50000 Dalton fragmentiert und diese einzeln nach Stoffgruppen oder als Mischung, je nach Indikation, mit biologischen Wirkstoffen, insbesondere Regulationsfaktoren aus Zellen und Geweben, wie auch anderen Pharmaka, einzeln oder in Kombination konjugiert und das $p_H$ durch Puffersubstanzen oder alkalisierenden Salzen auf 7,1 bis 7,8 eingestellt werden.

2. Verwendung von Polypeptiden und Peptiden nach Anspruch 1, dadurch gekennzeichnet, daß diese folgende Aminosäuren enthalten: Alanin, Asparaginsäure, Glutaminsäure, Phenylalanin, Thyrosin, Tryptophan, Ornithin, Lysin, Cystein, Cystin, Methionin.

0164423

3. Bevorzugte biologische Wirk- und Regulationsfaktoren sowie Pharmaka zur Konjugation an die
Träger- und Begleitstoffe, gekennzeichnet durch
die Verwendung von Organ- und Pflanzenextrakten,
pflanzlichen und tierischen Hormonen, insbesondere Wachstums-, Stoffwechsel- und Gewebehormonen,
Antihormonen, Überträgerstoffen, z. B. Neurotransmitter, Biological Response Modifiers, Abwehrstimulatoren, Paramunitätsinducern, Redox-
und Energieüberträgersystemen, z. B. NAD, NADP
und ATP, Enzymen, Antienzymen, Vitaminen, Chemotherapeutika einschließlich zytostatischen und
zytotoxischen Substanzen, oberflächenaktiven
Substanzen, Puffersubstanzen und gegebenenfalls
Bikarbonate, -Phosphate oder -Zitrate von Natrium,
Kalium, Magnesium, Mangan, Aluminium, Spurenelementen u. a.

4. Verwendung der Präparationen nach Anspruch 1 - 3
gegen Umweltsschäden von Bäumen und Pflanzen in
Form von Trockenpulvern oder wässrigen Lösungen.

5. Verwendung der Präparationen zur Verbesserung
der Futterverwertung und der Gewichtszunahme
von Nutztieren.

6. Verwendung der Präparationen zur Prophylaxe und
Therapie von Schäden durch ionisierende Strahlen
uns zytotoxische Substanzen.

7. Verwendung der Präparationen im Nährmedium von
Bakterien-, Zell- und Gewebekulturen.